# EUROPEAN PATENT APPLICATION

(11) **EP 4 474 794 A1**
(43) Date of publication of application: **11.12.2024**
(21) Application number: 22925001.4
(22) Date of filing: 14.12.2022
(51) Int. Cl.: G01N 5/02

(54) **INFORMATION PROCESSING DEVICE, PROGRAM, AND DETECTION DEVICE**

(30) Priority: 02.02.2022 JP 2022015168
(71) Applicant: Revorn Co., Ltd., Tokyo 104-0033 (JP)
(72) Inventor: MATSUOKA, Hiroaki, Tokyo 107-0061 (JP)
(74) Representative: Winter, Brandl - Partnerschaft mbB
(86) International application number: PCT/JP2022/045963
(87) International publication number: WO 2023/149097

(57) **Abstract**

[Problem] To provide an information processing device, a program, and a detection device that enable easy odor determination. [Solution] One aspect of the present invention provides an information processing device. This information processing device comprises a presentation unit and a provision unit. The presentation unit presents templates to information processing terminals in a selectable form. The templates include parameters for instructing an operation of a sensor at odor detection. The provision unit provides one of the templates, selected from an information processing terminal, to the information processing terminal.

## Description

### BACKGROUND

The present invention relates to an information processing apparatus, a program and a detection apparatus.

### RELATED ART

In general, an odor is not something that can be confirmed visually, but techniques have been proposed to visualize it (see, for example, Patent Document 1). Furthermore, techniques for identifying objects by an odor have also been proposed (see, for example, Patent Document 2).

### PRIOR ART DOCUMENTS

### Patent document

[Patent Document 1] JP2021-76458 A
[Patent document 2] WO2020/116490

### SUMMARY

### Problems to be Solved by Invention

By the way, when visualizing an odor, the corresponding odor may be expressed in words. In this case, by limiting the words used to a certain range, the odor can be accurately expressed. In addition, if a sensor or the like that detects an odor can be set appropriately in accordance with the range, the determination of an odor will be accurate.

In view of the above circumstances, the present invention provides an information processing apparatus, a program and a detection apparatus, which can easily perform determination of an odor.

### Means for Solving Problems

According to an aspect of the present invention, an information processing apparatus is provided. This information processing apparatus comprises a presentation unit and a provision unit. The presentation unit is configured to present templates to an information processing terminal in a selectable manner. Each of the templates includes a parameter that directs an operation of a sensor at a time of odor detection. The provision unit is configured to provide the information processing terminal with a template selected from the information processing terminal among the templates.

According to one aspect of the present invention, it is possible to easily perform determination of an odor.

### BRIEF DESCRIPTION OF DRAWINGS

FIG. 1 shows an example of a configuration of a system 100 according to an embodiment of the present invention.
FIG. 2 shows a configuration of an information processing apparatus 1.
FIG. 3 shows a configuration of an information processing terminal 3.
FIG. 4 shows a configuration of a detection apparatus 4.
FIG. 5 shows a configuration of a detection apparatus 5.
FIG. 6 is a block diagram showing a functional configuration of the system 100.
FIG. 7 is a diagram showing an example of a registration screen displayed on a browser of an information processing terminal 6 when a template is registered.
FIG. 8 is an activity diagram showing a flow of operation of the system 100.
FIG. 9 is a diagram showing a display example of a template.
FIG. 10 is a diagram showing a display example of a screen for selecting a sensor.
FIG. 11 is a diagram showing a display example of a determination result.
FIG. 12 is an activity diagram showing the flow of operation of the system 100 at the time of template registration.
FIG. 13 is an activity diagram showing the flow of operation of the system 100 when a template is registered.
FIG. 14 shows an example of a screen for selecting odor data.
FIG. 15 is a diagram for explaining an operation example of the system 100.

### DETAILED DESCRIPTION

Hereinafter, an embodiment of the present invention will be described with reference to drawings. Various features described in the embodiment below can be combined with each other.

A program for realizing a software in the present embodiment may be provided as a non-transitory computer readable storage medium that can be read by a computer, may be provided for download from an external server, or may be provided in such a manner that the program can be activated on an external computer to realize function thereof on a client terminal (so-called cloud computing).

A term "unit" in the present embodiment may include, for example, a combination of a hardware resource implemented as circuits in a broad sense and information processing of software that can be concretely realized by the hardware resource. Furthermore, various kinds of information are described in the present embodiment, and such information may be represented by, for example, physical values of signal values representing voltage and current, high and low signal values as a set of binary bits consisting of 0 or 1, or quantum superposition (so-called qubits), and communication and computation may be executed on a circuit in a broad sense.

The circuit in a broad sense is a circuit realized by properly combining at least a circuit, circuitry, a processor, a memory, and the like. In other words, a circuit includes an application specific integrated circuit (ASIC), a programmable logic device (e.g., simple programmable logic device (SPLD), a complex programmable logic device (CLPD), field programmable gate array (FPGA), and the like.

### Overall configuration

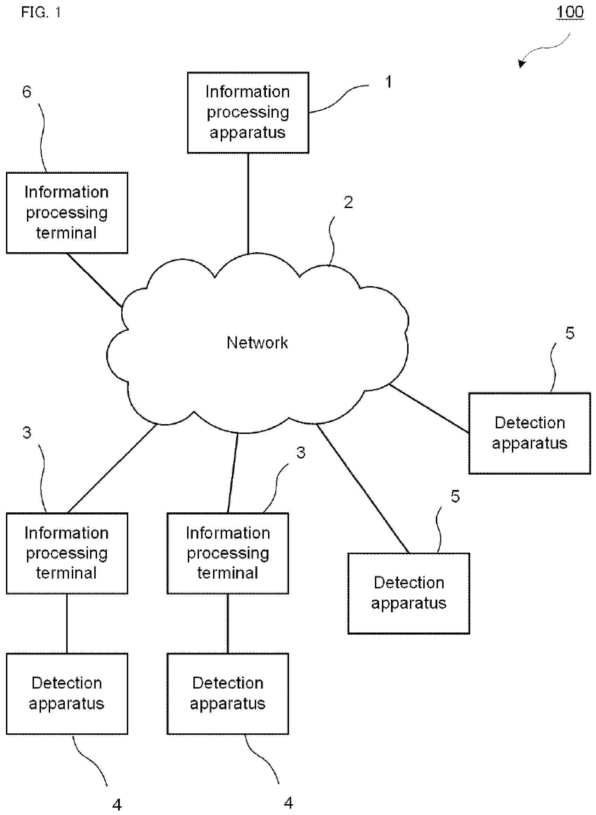

FIG. 1 shows an example of a configuration of a system 100 according to an embodiment of the present invention. As shown in the drawing, the system 100 is configured by connecting an information processing apparatus 1 to a network 2. An information processing terminal 3 is connected to the network 2, and a detection apparatus 4 is connected to the information processing terminal 3. Furthermore, a detection apparatus 5 and an information processing terminal 6 are connected to the network 2. There is no limit to number of information processing terminals 3, number of detection apparatuses 5, and number of information processing terminals 6, which are connected to the network 2, and at least one of them, including the information processing terminal 3 and the detection apparatus 5, only needs to be connected to the network 2. The information processing terminal 3, the detection apparatus 5, and the information processing terminal 6 may be connected to the network 2 as necessary and need not be always connected.

The information processing apparatus 1 is configured to provide a template including parameters, etc. that direct operations of sensors included in the detection apparatus 4 and the detection apparatus 5, and to perform determination of the odor detected by the detection apparatus 4 and the detection apparatus 5. The network 2 enables communication between the information processing apparatus 1 and the information processing terminal 3 or the detection apparatus 5, and includes, for instance, the Internet. The information processing terminal 3 is a tablet, smartphone or portable computer, and communicates with the information processing apparatus 1 and controls the detection apparatus 4. The detection apparatus 4 includes a sensor and performs detection of an odor. The detection apparatus 5, same as the detection apparatus 4, is configured to perform detection of an odor and communicate with the information processing apparatus 1 without going through an information processing terminal. The information processing terminal 6 is a computer or the like and is configured to set templates or the like as described below.

### 2. Configuration of information processing apparatus 1

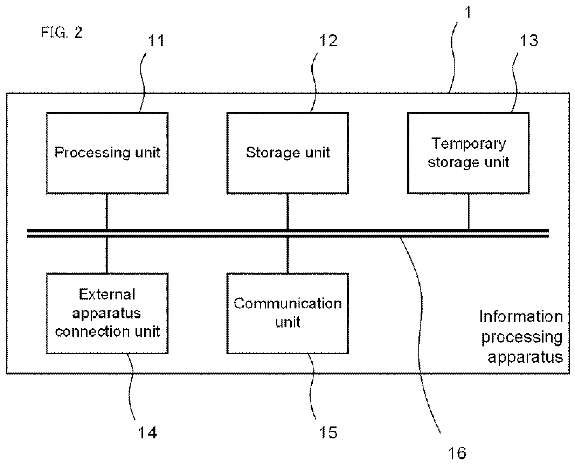

Next, a configuration of the information processing apparatus 1 will be described. FIG. 2 shows a configuration of the information processing apparatus 1. As shown in the drawing, the information processing apparatus 1 comprises a processing unit 11, a storage unit 12, a temporary storage unit 13, an external apparatus connection unit 14, and a communication unit 15, which are electrically connected via a communication bus 16 within the information processing apparatus 1.

The processing unit 11 is realized by a central processing unit (CPU), for example, and operates in accordance with a predetermined program stored in the storage unit 12 to realize various functions.

The storage unit 12 is a nonvolatile storage medium that stores various information. This is realized, for example, by a storage device such as a hard disk drive (HDD) or a solid state drive (SSD). The storage unit 12 may be arranged in another apparatus communicable with the information processing apparatus 1.

The temporary storage unit 13 is a volatile storage medium. This is realized by a memory such as random access memory (RAM), for example, and temporarily stores necessary information (arguments, array, etc.) when the processing unit 11 operates.

The external apparatus connection unit 14 is a connection unit in compliance with standard such as universal serial bus (USB) or high-definition multimedia interface (HDMI (registered trademark)), and allows connection of an input apparatus such as a keyboard or a display device such as a monitor.

The communication unit 15 is a communication means in compliance with, for instance, a local area network (LAN) standard, and realizes communication between the information processing apparatus 1 and the network 2 such as a local area network or the Internet via the network.

In addition, the information processing apparatus 1 may utilize a computer or a personal computer for general purpose server, and the information processing apparatus 1 may be configured by utilizing two or more computers.

### 3. Configuration of information processing terminal 3

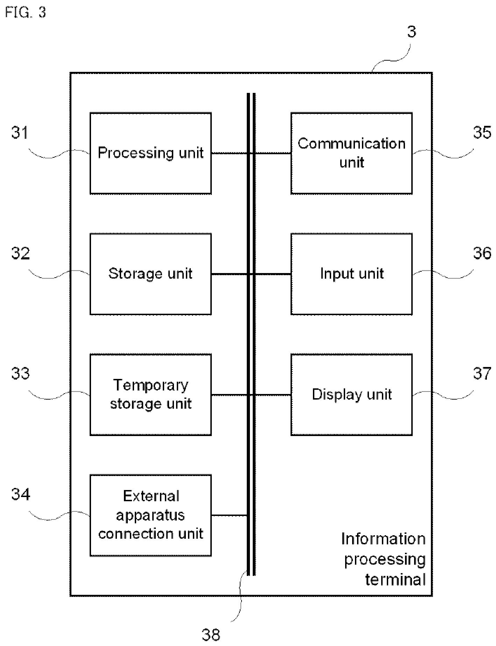

FIG. 3 shows a configuration of the information processing terminal 3. As shown in the drawing, the information processing terminal 3 comprises a processing unit 31, a storage unit 32, a temporary storage unit 33, an external apparatus connection unit 34, a communication unit 35, an input unit 36 and a display unit 37, which are electrically connected via a communication bus 38 within the information processing terminal 3.

The processing unit 31 is realized by a CPU, for example, and operates in accordance with a predetermined program stored in the storage unit 32 to realize various functions.

The storage unit 32 is a nonvolatile storage medium that stores various information.

The temporary storage unit 33 is a volatile storage medium. This is realized by a memory such as a random access memory, for instance, so that necessary information (arguments, arrays, etc.) is temporarily stored when the processing unit 21 operates.

The external apparatus connection unit 34 is a connection unit in compliance with standard such as universal serial bus or Bluetooth (registered trademark), for example, and may allow connection of the detection apparatus 4 or the like.

The communication unit 35 is a communication means in compliance with, for instance, a local area network (LAN) standard, and realizes communication between the information processing apparatus 1 and the network 2 such as a local area network or the Internet via the network. Further, the communication unit 35 includes a communication means capable of communicating through a cellular phone network.

The input unit 36 is configured to receive an operation input. The display unit 37 is configured to display information or the like on a screen. The input unit 36 and the display unit 37 may be integrated as a touch panel.

A general-purpose smartphone, a tablet terminal, a notebook PC, etc. can be utilized for the information processing terminal 3.

### 4. Configuration of detection apparatus 4

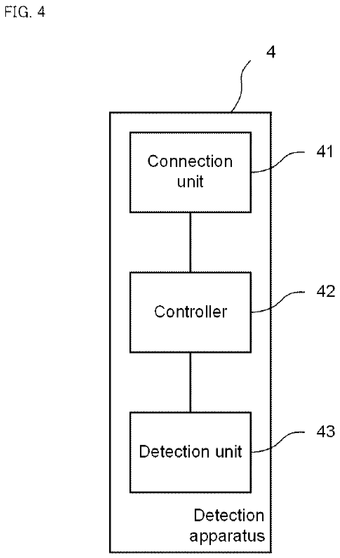

FIG. 4 shows a configuration of the detection apparatus 4. As shown in the drawing, the detection apparatus 4 comprises a connection unit 41, a controller 42, and a detection unit 43.

The connection unit 41 is in compliance with standard such as universal serial bus or Bluetooth (registered trademark), for instance, and allows connection of the information processing terminal 3, etc. The controller 42 controls operation of the detection unit 43 and transmit/receive information with the information processing terminal 3 and the like. The detection unit 43 includes a sensor that detects odors. The sensor included in the detection unit 43 is, for example, a sensor that detect gas such as carbon dioxide, carbon monoxide, methane, butane, ammonia, etc. or two or more Quartz Crystal Microbalance sensors, each Quartz Crystal Microbalance sensor has a quartz crystal formed by a thin film having non-specific adsorption properties, and each quartz oscillator has a different compound deposited on it. The deposited compound is, for instance, D-phenylalanine, D-tyrosine, DL-histidine, D-glucose, adenine, polyethylene, etc. For any one of these compounds, resonance frequency changes due to adhesion of odor component, and since degree of adhesion of the odor component varies from compound to compound, each of the Quartz Crystal Microbalance sensors can detect different odors.

### 5. Configuration of detection apparatus 5

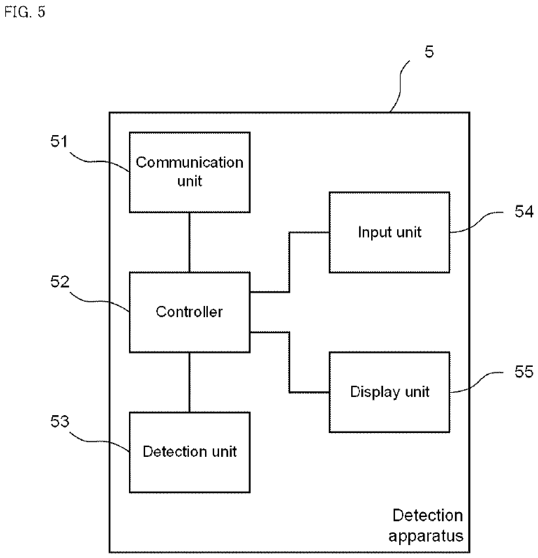

FIG. 5 shows a configuration of the detection apparatus 5. As shown in the drawing, the detection apparatus 5 comprises a communication unit 51, a controller 52, a detection unit 53, a display unit 55 and an input unit 54.

The communication unit 51 is a communication means in compliance with, for instance, a local area network (LAN) standard, and realizes communication between the detection apparatus 5 and the network 2 such as a local area network or the Internet via the network. Further, the communication unit 51 may include a communication means capable of communicating through a cellular phone network.

The controller 52 controls operation of the detection unit 53 and transmits/receives information with the information processing apparatus 1 or the like. The detection unit 53, same as the detection unit 43, includes a sensor that detects odors.

The input unit 54 is configured to receive an operation input. The display unit 55 is configured to display information or the like on a screen. The input unit 54 and the display unit 55 may be integrated as a touch panel.

### 6. Configuration of information processing terminal 6

Since the configuration of the information processing terminal 6 is the same as that of the information processing apparatus 1, a detailed explanation is omitted.

### 7. Functional configuration of system 100

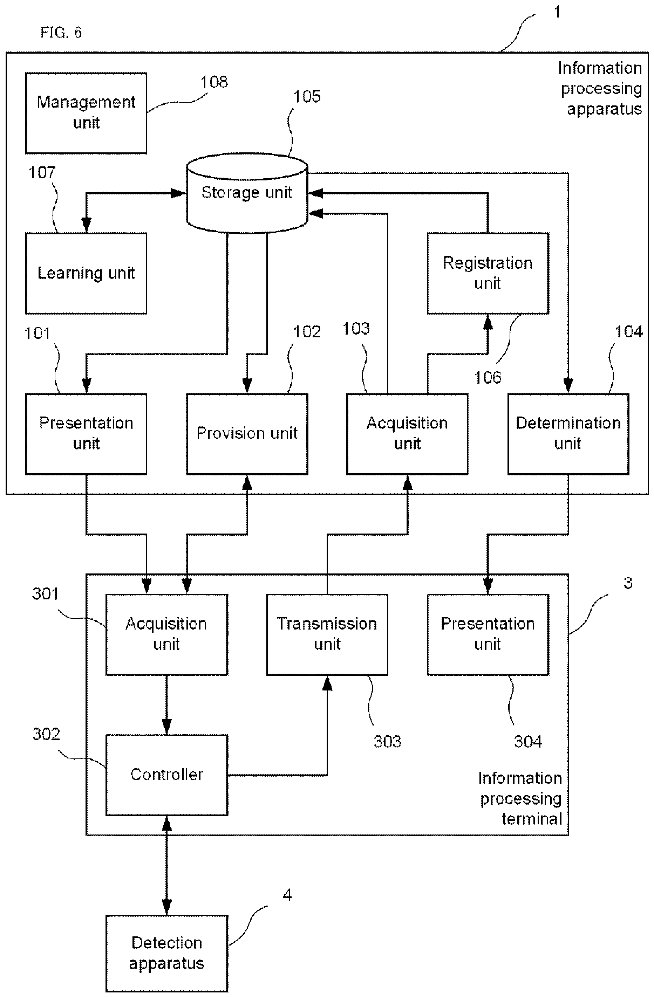

Next, function of the system 100 will be described. FIG. 6 is a block diagram showing a functional configuration of the system 100. Here, an example in which the system 100 comprises the information processing apparatus 1, the information processing terminal 3, and the detection apparatus 4 will be described, and the detection apparatus 5 has the same configuration as the information processing terminal 3 and the detection apparatus 4 combined.

As shown in FIG. 6, the information processing apparatus 1 comprises a presentation unit 101, a provision unit 102, an acquisition unit 103, a determination unit 104, a storage unit 105, a registration unit 106, a learning unit 107, and a management unit 108. Each of these functional units is realized by a program that allows the computer to function as an information processing apparatus, and specifically, is realized by the processing unit 11 operating in accordance with the program, and during the operation, the processing unit 11 allows the storage unit 12, the temporary storage unit 13, the external apparatus connection unit 14 and the communication unit 15 to operate as necessary.

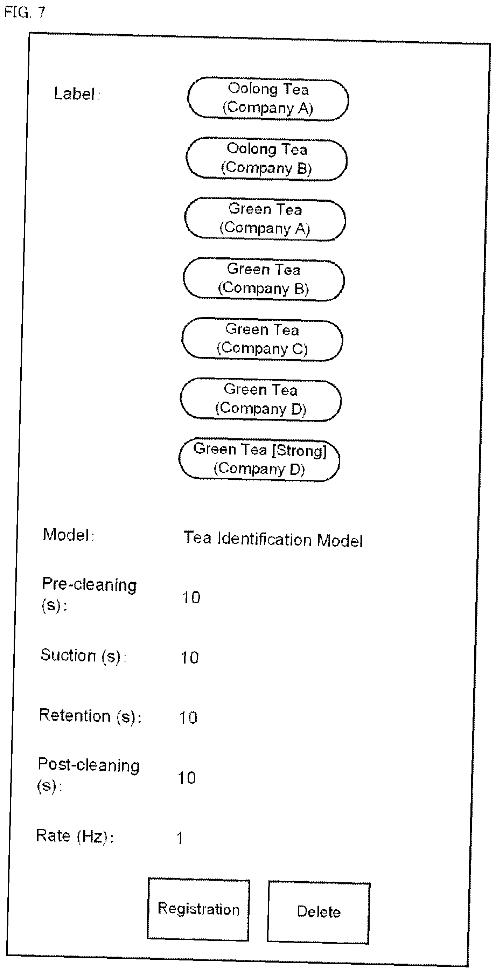

The presentation unit 101 presents templates to the information processing terminal 3 in a selectable manner. Each of the templates includes directions or the like for allowing the detection apparatus 4 to operate appropriately and is registered by the information processing terminal 6 or the like. The information processing terminal 6 accesses the information processing apparatus 1 with a browser, etc., and registers a template. FIG. 7 is a diagram showing an example of a registration screen displayed on the browser of the information processing terminal 6 when a template is registered. As shown in the drawing, on the registration screen of the template, labels, a determination model, and parameters can be registered.

The label included in the template expresses an odor in words, and the determination model is a learned model that has machine-learned a relationship between an output of the sensor and an odor in advance. The determination model is used for performing determination in the determination unit 104, and the template includes a specification of the determination model. In addition, the parameters include, for example, the specification of each time of pre-cleaning, suction, retention, and post-cleaning, as well as the specification of a rate that is a sampling rate of the sensor. The pre-cleaning, suction, retention, and post-cleaning are operations of the sensor, respectively. Thus, each of the templates includes a parameter that directs an operation of the sensor at a time of odor detection. Each of the templates may also include a specification of the detection apparatus 4, i.e., the specification of the sensor, used for detection.

The provision unit 102 is configured to provide the information processing terminal 3 with the template selected from the information processing terminal 3 among the templates presented by the presentation unit 101.

The acquisition unit 103 is configured to acquire a detection result detected by the sensor of the detection apparatus 4 from the information processing terminal 3.

The determination unit 104 is configured to perform determination of an odor based on the detection result using the determination model. The determination unit 104 indicates a result of the determination with a percentage of odor content corresponding to the label, and presents the result to the information processing terminal 3.

The storage unit 105 is configured to store the templates and the determination model and store the detection result acquired by the acquisition unit 103. The detection result may be stored temporarily and deleted when the determination by the determination unit 104 is completed.

The registration unit 106 is configured to receive specifications of parameters and the determination model from the information processing terminal 3 and newly register a template based on the received specifications. When the registration unit 106 registers a template, the information processing terminal 3 is operated by a provider of the template.

The learning unit 107 is configured to perform machine learning based on words to be used as a label and a plurality of detection results corresponding to the words, and generate a determination model based on a result of the machine learning. The machine learning when generating a determination model is performed, for example, by using the technology described in Patent Document 2.

The management unit 108 is configured to manage approval or disapproval of use by the information processing terminal 3 and charging information. The charging information includes a usage fee of the template registered by the information processing terminal.

The information processing terminal 3 comprises an acquisition unit 301, a controller 302, a transmission unit 303, and a presentation unit 304. Each of these functional units is operated by the processing unit 31 in accordance with a program, and during the operation, each of the functional units is realized by the processing unit 31 that allows the storage unit 32, the temporary storage unit 33, the external apparatus connection unit 34, the communication unit 35, the input unit 36, and the display unit 37 to operate as necessary.

The acquisition unit 301 is configured to acquire a template from the information processing apparatus 1. The controller 302 is configured to control the operation of the sensors of the detection apparatus 4 based on the parameters included in the template acquired by the acquisition unit 301. The transmission unit 303 is configured to transmit the detection result of the odor by the sensor of the detection apparatus 4 to the information processing apparatus 1. The presentation unit 304 is configured to acquire and present the determination result determined by the information processing apparatus 1 based on the detection result transmitted by the transmission unit 303.

Although not shown in drawing, the detection apparatus 5 includes a functional unit in which the information processing terminal 3 and the detection apparatus 4 are combined, and for example, the detection apparatus 5 includes an acquisition unit, an odor detection unit, a transmission unit, and a presentation unit. This acquisition unit (not shown) acquires a template from the information processing apparatus 1 in the same manner as the acquisition unit 301. The odor detecting unit (not shown) is a functional unit combining the functions of the controller 302 and the detection apparatus 4, and performs detection of an odor by operating based on the parameters. The transmission unit (not shown) transmits a detection result of an odor by the odor detection unit (not shown) to the information processing apparatus 1 in the same manner as the transmission unit 303. The presentation unit (not shown) presents a determination result determined by the information processing apparatus 1 based on the detection result in the same manner as the presentation unit 304.

### 8. Operation of system 100

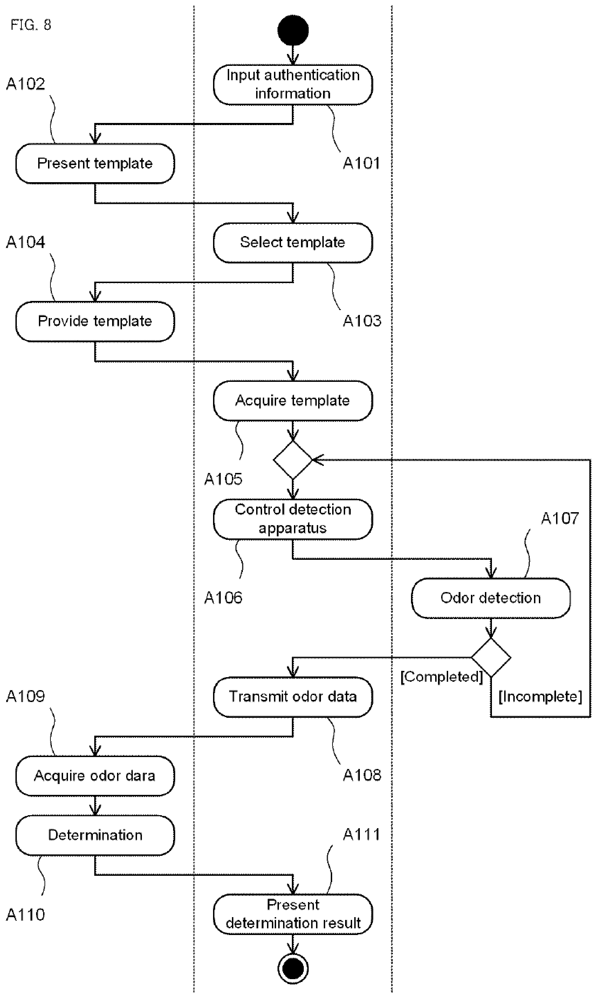

Next, the operation of the system 100 is described. FIG. 8 is an activity diagram showing a flow of operation of the system 100.

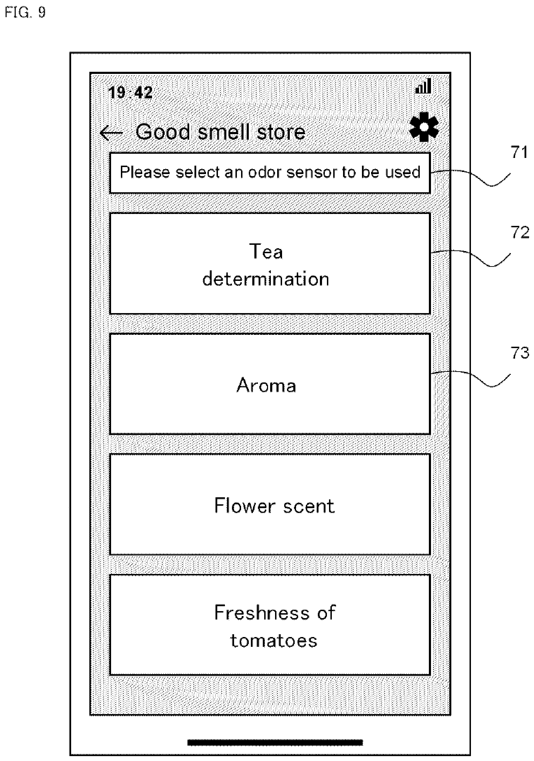

First, when the information processing terminal 3 accesses the information processing apparatus 1 by inputting authentication information, etc. (A101), the presentation unit 101 of the information processing apparatus 1 presents to the information processing terminal 3 available templates in a selectable manner (A102). The templates to be presented may be all templates or may be a template selected according to the input authentication information. The template is presented to the information processing terminal 3, for example, in the manner shown in FIG. 9. FIG. 9 is a diagram showing a display example of a template.

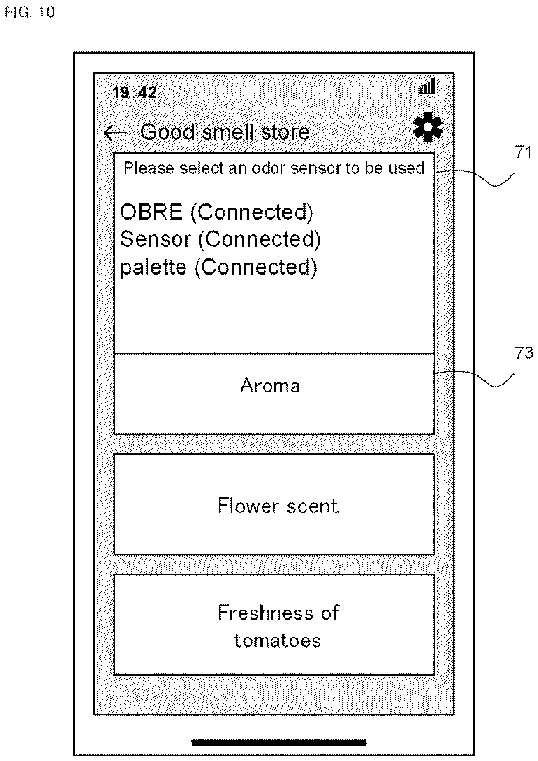

As shown in FIG. 9, the presentation of the template is performed as an object 71 for selecting the sensor to be used, an object 72 and an object 73 for selecting the template. When the object 71 is selected, it becomes possible to select a sensor to be used, that is, the type of detection apparatus 4, as shown in FIG. 10. FIG. 10 is a diagram showing a display example of a screen for selecting a sensor. The object for selecting the template may be changed according to the operation of the object 71, that is, according to the type of the selected sensor. This is because there may be sensors that cannot be used depending on the contents of the template.

When any of the templates is selected (A 103) while the templates are being presented in a selectable state, the information processing apparatus 1 receives this selection, and the provision unit 102 provides the information processing terminal 3 with the template (A 104). For example, when the template with a name such as "tea determination" is selected by the operation of the object 72, the provision unit 102 acquires a template "tea determination" from the storage unit 105 and provides it to the information processing terminal 3. Then, the acquisition unit 301 of the information processing terminal 3 acquires the template (A105).

Next, the controller 302 of the information processing terminal 3 controls the detection apparatus 4, that is, the sensor that detects odors, according to the template (A106). In response to this, the detection apparatus 4 performs odor detection (A107). Since odor detection includes, for example, pre-cleaning, suction, retention, and post-cleaning processes, the detection apparatus performs odor detection until all of these processes are completed (A107).

When the odor detection by the detection apparatus 4 is completed, the transmission unit 303 of the information processing terminal 3 transmits a detection result to the information processing apparatus 1 (A108). The acquisition unit 103 of the information processing apparatus 1 acquires the detection result and the storage unit 105 is allowed to store the detection result (A109).

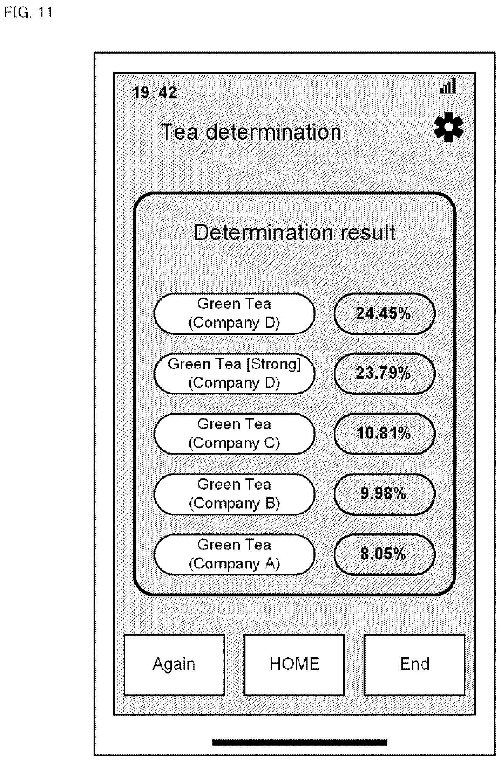

Subsequently, the determination unit 104 of the information processing apparatus 1 performs determination of an odor (A110) using the learned model specified in the template, the determination result is notified to the information processing terminal 3. In the information processing terminal 3, the presentation unit 304 presents the determination result. The presentation of the determination result is configured, for example, as shown in FIG. 11. FIG. 11 is a diagram showing a display example of the determination result. As shown in the drawing, the determination result is obtained by using the labels included in the template and presenting the ratio (percentage) of the odor represented by each label. This completes the series of operations.

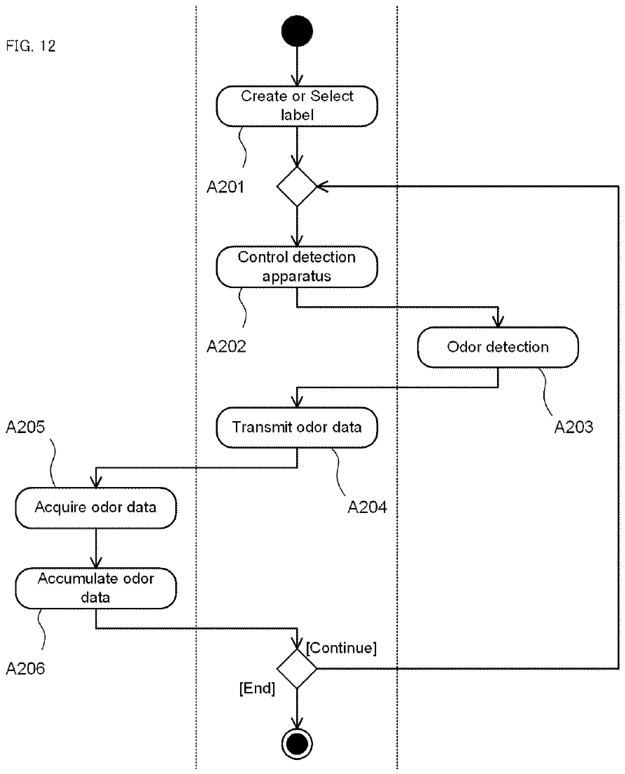

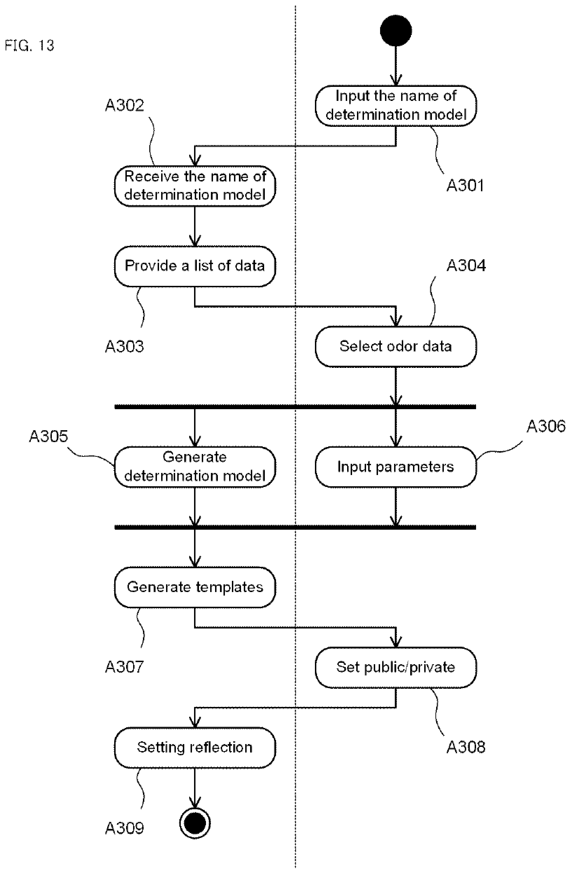

Next, the operation at the time of template registration will be described. FIG. 12 and FIG. 13 are activity diagrams showing the flow of operation of the system 100 at the time of template registration.

When the template is registered, first, odor data is registered from the information processing terminal 3. At this time, in the information processing terminal 3, a label is created or selected first (A201). In the case where this label corresponds to the odor date to be registered, for example, in the case where the odor data that has detected the odor of the green tea of Company A is registered, the label of "Green tea (Company A)" is selected, and in the case where such a label does not exist, a label is created.

Next, the control unit 302 of the information processing terminal 3 controls the detection apparatus 4 (A202), and accordingly, the detection apparatus 4 performs odor detection (A203). When the odor detection by the detection apparatus 4 is completed, the transmission unit 303 of the information processing terminal 3 transmits the detection result to the information processing apparatus 1 (A204). The acquisition unit 103 of the information processing apparatus 1 acquires the detection result (A205) and the information processing apparatus 1 allows the storage unit 105 to store and accumulate it (A205). In the information processing terminal 3, the registration of odor is repeated several times, and when these operations are completed, the process ends.

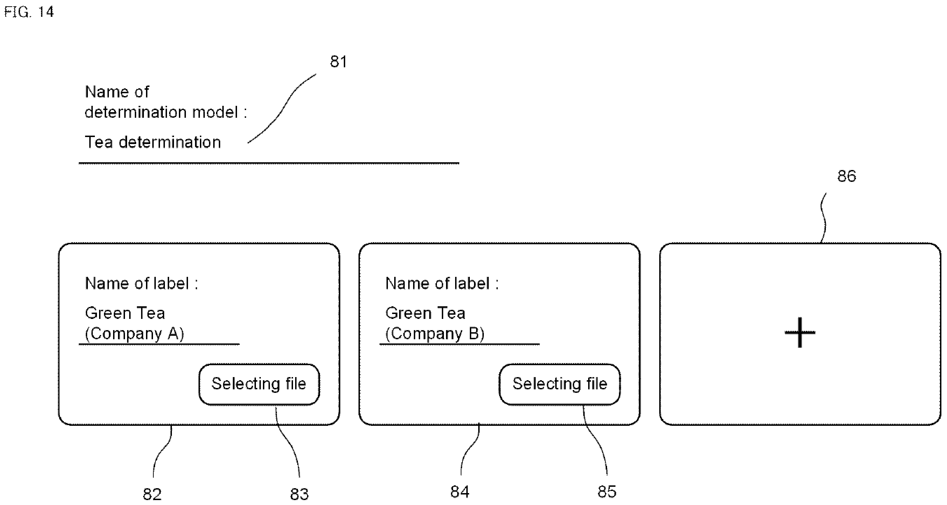

Next, the template registration is described. When the template is registered, the information processing terminal 3 or the information processing terminal 6 inputs the name of the determination model (A310). The registration unit 106 receives this input (A302). Then, the registration unit 106 provides the information processing terminal 3, etc. with a list of available odor data (A303). In the information processing terminal 3, etc., the odor data to be used is selected (A304). The available odor data is one accumulated by the information processing terminal 3 or the like, or one that is open to the public, among the odor data that can be accumulated in the storage unit 105. The list of available odor data is displayed, for example, like the screen as shown in FIG. 14. FIG. 14 shows an example of the screen for selecting odor data. This screen displays an object 81 that specifies the name of the determination model, an object 82 and an object 84 that indicate the label to be used. The object 82 and the object 84 include an object 83 and an object 85 for selecting files, respectively. The files indicate odor data, and when the object 83 or the object 85 is selected, the available files of the odor data are displayed in a selectable manner. In addition, an object 86 indicates an addition of a label.

When the odor data to be used is selected, the learning unit 107 performs machine learning based on the selected odor data and create a determination model (A305) in the information processing apparatus 1. At this time, the information processing terminal 3, etc., inputs parameters other than the determination model required for the template (A306). Thereafter, the learning unit 107 generates a template including the specification of the determination model and parameters (A307). Subsequently, the information processing terminal 3, etc., sets the created template to be public or private (A308), and accordingly, the registration unit 106 sets the created template to be public or private (A309). Setting the template to be public or private means that the person who created the template sets whether or not to allow others to use the template. At this time, it is also possible to set whether or not to charge for the use of the template by others.

### 9. Example of operation of system 100

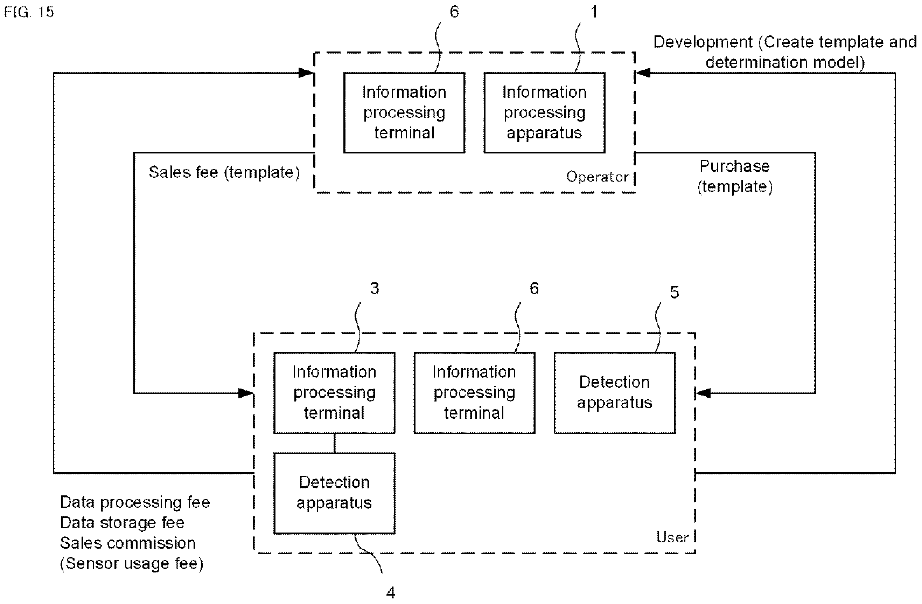

Next, an operation example of the system 100 will be described. FIG. 15 is a diagram for explaining an operation example of the system 100. As shown in the drawing, the operator performs development work such as managing the information processing apparatus 1 and creating templates by using the information processing apparatus 1 and the information processing terminal 6. On the other hand, the user side purchases a template and performs odor determination, and also performs development work such as creating a template and creating a determination model by himself/herself by using the information processing terminal 3, the detection apparatus 4, the detection apparatus 5, and the information processing terminal 6. In the case where the template or the like created by the user during the development work is open to the public on a chargeable basis, a charge may be collected from the user who uses it. When the template that is open to the public by the user on a chargeable basis is purchased by another user, the operator collects the sales fee from the user who used the template and pays it to the user who developed it, and collects the sales commission, data processing fee, data storage fee, etc. from the user. In addition, in the case where the operator has an ownership right of the detection apparatus 4 and the detection apparatus 5 that are used by the user, the operator may collect a usage fee of the detection apparatus4 and detection apparatus5 as a sensor usage fee.

### 10. Others

The present invention may be provided in each of the following aspects.
(1) An information processing apparatus, comprising: a presentation unit configured to present templates to an information processing terminal in a selectable manner, each of the templates including a parameter that directs an operation of a sensor at a time of odor detection; and a provision unit configured to provide the information processing terminal with a template selected from the information processing terminal among the templates.
(2) The information processing apparatus according to (1), comprising an acquisition unit configured to acquire a detection result of the sensor from the information processing terminal.
(3) The information processing apparatus according to (2), comprising a determination unit configured to perform determination of an odor based on the detection result using a determination model, the determination model being a learned model that has machine-learned a relationship between an output of the sensor and an odor in advance, wherein each of the templates includes a specification of the determination model.
(4) The information processing apparatus according to (3), wherein each of the templates includes a label that expresses an odor in words, and the determination unit indicates a result of the determination with a percentage of odor content corresponding to the label.
(5) The information processing apparatus according to (4), comprising a registration unit configured to receive specifications of the parameter and the determination model, and newly register the template based on the received specifications.
(6) The information processing apparatus according to (5), comprising, a learning unit configured to perform machine learning based on words to be used as a label and a plurality of the detection results corresponding to the words and generate the determination model based on a result of the machine learning.
(7) The information processing apparatus according to (6), comprising a management unit configured to manage approval or disapproval of use by the information processing terminal and charging information, wherein the charging information includes a usage fee of the template registered by the information processing terminal.
(8) The information processing apparatus according to any one of (1) to (7), wherein each of the templates includes a specification of the sensor.
(9) A program that allows a computer to operate as an information processing apparatus, wherein the program allows the computer to function as the information processing apparatus according to any one of claims 1 to 8.
(10) A program that allows a computer that is connected to a sensor to operate as an information processing terminal, wherein the information processing terminal comprises: an acquisition unit configured to acquires a template from an information processing apparatus, the template including a parameter that directs an operation of the sensor at a time of odor detection; a controller configured to control the operation of the sensor based on the parameter; a transmission unit configured to transmit a detection result of an odor by the sensor to the information processing apparatus; and a presentation unit configured to present a determination result determined by the information processing apparatus based on the detection result.
(11) A detection apparatus for detecting an odor, comprising: an acquisition unit configured to acquire a template from an information processing apparatus; an odor detection unit configured to detect an odor by operating based on a parameter included in the template, the parameter directing an operation of the odor detection unit at a time of odor detection; a transmission unit configured to transmit a detection result of an odor by the odor detection unit to the information processing apparatus; and a presentation unit configured to present a determination result determined by the information processing apparatus based on the detection result.

Of course, the present invention is not limited to the above aspects.

### REFERENCE SIGNS LIST

1: Information processing apparatus
2: Network
3: Information processing terminal
4: Detection apparatus
5: Detection apparatus
6: Information processing terminal
11: Processing unit
12: Storage unit
13: Temporary storage unit
14: External apparatus connection unit
15: Communication unit
16: Communication bus
21: Processing unit
31: Processing unit
32: Storage unit
33: Temporary storage unit
34: External apparatus connection unit
35: Communication unit
36: Input unit
37: Display unit
38: Communication bus
41: Connection unit
42: Controller
43: Detection unit
51: Communication unit
52: Controller
53: Detection unit
54: Input unit
55: Display unit
71: Object
72: Object
73: Object
81: Object
82: Object
83: Object
84: Object
85: Object
86: Object
100: System
101: Presentation unit
102: Provision unit
103: Acquisition unit
104: Determination unit
105: Storage unit
106: Registration unit
107: Learning unit
108: Management unit
301: Acquisition unit
302: Controller
303: Transmission unit
304: Presentation unit

## Claims

1. An information processing apparatus, comprising:
a presentation unit configured to present templates to an information processing terminal in a selectable manner,
each of the templates including a parameter that directs an operation of a sensor at a time of odor detection; and
a provision unit configured to provide the information processing terminal with a template selected from the information processing terminal among the templates.

2. The information processing apparatus according to claim 1, comprising
an acquisition unit configured to acquire a detection result of the sensor from the information processing terminal.

3. The information processing apparatus according to claim 2, comprising
a determination unit configured to perform determination of an odor based on the detection result using a determination model,
the determination model being a learned model that has machine-learned a relationship between an output of the sensor and an odor in advance, wherein
each of the templates includes a specification of the determination model.

4. The information processing apparatus according to claim 3, wherein
each of the templates includes a label that expresses an odor in words, and
the determination unit indicates a result of the determination with a percentage of odor content corresponding to the label.

5. The information processing apparatus according to claim 4, comprising
a registration unit configured to receive specifications of the parameter and the determination model, and newly register the template based on the received specifications.

6. The information processing apparatus according to claim 5, comprising,
a learning unit configured to perform machine learning based on words to be used as a label and a plurality of the detection results corresponding to the words and generate the determination model based on a result of the machine learning.

7. The information processing apparatus according to claim 6, comprising
a management unit configured to manage approval or disapproval of use by the information processing terminal and charging information, wherein
the charging information includes a usage fee of the template registered by the information processing terminal.

8. The information processing apparatus according to any one of claims 1 to 7, wherein
each of the templates includes a specification of the sensor.

9. A program that allows a computer to operate as an information processing apparatus, wherein
the program allows the computer to function as the information processing apparatus according to any one of claims 1 to 8.

10. A program that allows a computer that is connected to a sensor to operate as an information processing terminal, wherein
the information processing terminal comprises:
an acquisition unit configured to acquire a template from an information processing apparatus, the template including a parameter that directs an operation of the sensor at a time of odor detection;
a controller configured to control the operation of the sensor based on the parameter;
a transmission unit configured to transmit a detection result of an odor by the sensor to the information processing apparatus; and
a presentation unit configured to present a determination result determined by the information processing apparatus based on the detection result.

11. A detection apparatus for detecting an odor, comprising:
an acquisition unit configured to acquire a template from an information processing apparatus;
an odor detection unit configured to detect an odor by operating based on a parameter included in the template, the parameter directing an operation of the odor detection unit at a time of odor detection;
a transmission unit configured to transmit a detection result of an odor by the odor detection unit to the information processing apparatus; and
a presentation unit configured to present a determination result determined by the information processing apparatus based on the detection result.
